# FASCICULE DE BREVET EUROPEEN

(11) **EP 2 598 611 B1**
(45) Date de publication et mention de la délivrance du brevet: **30.04.2014**
(21) Numéro de dépôt: 11743260.9
(22) Date de dépôt: 24.06.2011
(51) Int. Cl.: C10G 45/08, C10G 45/10, B01J 23/85, B01J 27/185, B01J 27/19, B01J 31/02, B01J 31/22, B01J 37/02, C07C 1/32

(54) **PROCEDE D'HYDRODESULFURATION D'UNE COUPE ESSENCE EN PRESENCE D'UN CATALYSEUR SULFURE SUPPORTE PREPARE AU MOYEN D'AU MOINS UN OLIGOSACCHARIDE CYCLIQUE**
VERFAHREN ZUR HYDROENTSCHWEFELUNG EINER BENZINFRAKTION UNTER VERWENDUNG EINES AUS MINDESTENS EINEM ZYKLISCHEN OLIGOSACCHARID HERGESTELLTEN GETRÄGERTEN SULFID-KATALYSATORS
METHOD FOR THE HYDRODESULFURIZATION OF A GASOLINE FRACTION IN THE PRESENCE OF A SUPPORTED SULFIDE CATALYST PREPARED USING AT LEAST ONE CYCLIC OLIGOSACCHARIDE

(30) Priorité: 29.07.2010 FR 1003191
(43) Date de publication de la demande: 05.06.2013
(73) Titulaire: IFP Energies Nouvelles, 92852 Rueil Malmaison Cedex (FR)
(72) Inventeur: DIEHL, Fabrice, F-69007 Lyon (FR); DEVERS, Elodie, F-69007 Lyon (FR)
(86) Numéro de dépôt international: PCT/FR2011/000366
(87) Numéro de publication internationale: WO 2012/022848

(56) Documents cités:
- WO-A1-96/41848
- WO-A2-2007/084438
- CARO ET AL: "Enhancement of dibenzothiophene biodesulfurization using beta-cyclodextrins in oil-to-water media", FUEL, IPC SCIENCE AND TECHNOLOGY PRESS, GUILDFORD, GB, vol. 86, no. 16, 9 octobre 2007 (2007-10-09), pages 2632-2636, XP022293310, ISSN: 0016-2361, DOI: DOI:10.1016/J.FUEL.2007.02.033

## Description

### Domaine de l'invention

La présente invention se rapporte au domaine des hydrotraitements de charges hydrocarbonées contenant du soufre, de préférence de type essence et notamment de type essence de craquage catalytique. Plus précisément, elle se rapporte à l'hydrodésulfuration de coupes essences dont la teneur en soufre a besoin d'être diminuée de manière à valoriser lesdites coupes dans le pool essence tout en limitant la perte d'indice d'octane.

### Art antérieur

Le durcissement des normes de pollution automobile en 2009 dans la communauté européenne contraint les raffineurs à réduire très fortement la teneur en soufre dans les gazoles et les essences, au maximum à 10 parties par million poids (ppm) de soufre dans les gazoles et les essences au 1er janvier 2009, contre 50 ppm depuis le 1er janvier 2005 (mesurée par la méthode ASTM D-4294). Ces normes concernent non seulement la teneur totale en soufre mais également la nature des composés soufrés tels que les mercaptans. Ces contraintes se traduisent par un besoin de nouvelles unités de raffinage ou bien par une forte augmentation de l'activité à iso-volume des catalyseurs d'hydrotraitement. D'autre part, ces nouvelles normes sont également accompagnées de contrainte en terme de qualité produit, comme par exemple pour les essences d'un haut indice d'octane.

La production d'essences reformulées, c'est-à-dire issues de procédés d'hydrotraitement, répondant aux nouvelles normes d'environnement nécessite notamment que l'on diminue le moins possible leur concentration en mono-oléfines afin de conserver un indice d'octane élevé, mais que l'on diminue de façon importante leur teneur en soufre. Les essences de craquage catalytique (FCC), qui peuvent représenter 30 à 50 % volumique du pool essence, présentent des teneurs en oléfines et en soufre élevées. Le soufre présent dans les essences reformulées est imputable, à près de 90 % poids, à l'essence craquage catalytique. La désulfuration (l'hydrodésulfuration) des essences et principalement des essences de craquage catalytique (FCC) est donc d'une importance évidente pour le respect des spécifications. L'hydrotraitement (ou hydrodésulfuration) des essences de craquage catalytique, lorsqu'il est réalisé dans des conditions classiques connues de l'homme du métier permet de réduire la teneur en soufre de la coupe. Cependant, ce procédé présente l'inconvénient majeur d'entraîner une chute très importante de l'indice d'octane de la coupe, en raison de la saturation de l'ensemble des oléfines au cours de l'hydrotraitement. Il a donc été proposé des procédés permettant de désulfurer profondément les essences de craquage catalytique tout en maintenant l'indice d'octane à un niveau élevé, c'est à dire en maintenant une bonne sélectivité (ratio entre hydrodésulfuration et hydrogénation des oléfines). Par exemple, le brevet US 5.318.690 propose un procédé consistant à fractionner l'essence, adoucir la fraction légère et à hydrotraiter la fraction lourde sur un catalyseur conventionnel puis à la traiter sur une zéolithe ZSM-5 pour retrouver approximativement l'indice d'octane initial.

En plus de la sélectivité, une autre performance catalytique que les raffineurs cherchent à améliorer est l'activité catalytique. Un moyen efficace pour augmenter l'activité des catalyseurs supportés est d'augmenter la quantité de phase active sous forme sulfure, ce qui se traduit préalablement par un dépôt maximal de la phase active sous forme oxyde associée à la surface du support. Toutefois, cette quantité maximale (habituellement déposée par imprégnation à sec) est limitée par les propriétés texturales du support et en particulier sa surface spécifique et son volume poreux. De plus, dans le cas particulier où le support utilisé comporte l'élément aluminium, cette concentration importante en phase oxyde déposée favorise la formation de phases oxydes cristallisées du type Al₂(MoO₄)₃, CoAl₂O₄, NiAl₂O₄, etc. qui s'avèrent être réfractaires à l'étape de sulfuration. Ceci se traduit logiquement par une perte indirecte de l'activité catalytique puisque toute la phase oxyde déposée n'est pas utilisée au maximum de son potentiel. D'autre part, une augmentation de la teneur en phase active peut conduire à la formation de cristallites de MoO₃, NiO, CoO, Co₃O₄ ou de CoMoO₄ de taille suffisamment importante pour être détectables en DRX. Ces espèces sont également connues pour diminuer le taux de sulfuration des catalyseurs d'hydrotraitement, et donc leurs performances.

La composition et l'utilisation des catalyseurs d'hydrotraitement et notamment des catalyseurs d'hydrodésulfuration sont particulièrement bien décrits dans l'article de B. S Clausen, H. T. Topsøe, et F. E. Massoth, issu de l'ouvrage Catalysis Science and Technology, 1996, volume 11, Springer-Verlag. Ainsi, ces catalyseurs comprennent généralement au moins un métal du groupe VIB et/ou au moins un métal du groupe VIII du tableau périodique des éléments. Les formulations les plus courantes sont de type cobalt-molybdène (CoMo), nickel-molybdène (NiMo) et nickel-tungstène (NiW). Ces catalyseurs peuvent se présenter sous forme massique ou bien à l'état supporté. Dans ce dernier cas, la matrice poreuse est généralement un oxyde amorphe ou mal cristallisé (alumine, silice-alumine, etc.) éventuellement associé à un tamis moléculaire zéolithique ou non zéolithique. Après préparation, lesdits catalyseurs se présentent souvent sous forme d'oxyde. Leur forme active et stable pour les procédés d'hydrotraitement et notamment pour les procédés d'hydrodésulfuration étant la forme sulfurée, ces catalyseurs sont soumis à une étape de sulfuration.

Cependant la dispersion de la phase active ou de ces précurseurs oxyde ou oxy-hydroxyde est directement liée à la surface spécifique offerte par le support : pour de fortes densités surfaciques en molybdène, la formation de phases réfractaires à la sulfuration par frittage a en effet été rapportée ; il s'agit par exemple dans le cas des catalyseurs CoMo des phases CoMoO₄ ou Co₃O₄ (B. S Clausen, H. T. Topsøe, et F.E. Massoth, issu de l'ouvrage Catalysis Science and Technology, volume 11 (1996), Springer-Verlag). De nouvelles techniques de préparation des catalyseurs ont besoin d'être développées pour améliorer encore les performances de ces catalyseurs et satisfaire les législations à venir.

En particulier, il convient de contrôler les interactions entre le support et les précurseurs de la phase active qui aboutissent à des espèces réfractaires à la sulfuration (par exemple, Al₂(MoO₄)₃, CoAl₂O₄ ou NiAl₂O₄), inutiles à la réaction catalytique et ayant des effets indésirables sur l'activité catalytique.

Ainsi tout moyen visant à limiter la formation de ces phases, soit réfractaires à la sulfuration, soit induisant une immobilisation des espèces sous une forme catalytiquement moins active, serait intéressant pour conduire à une amélioration significative des performances des catalyseurs d'hydrotraitement et notamment des catalyseurs d'hydrodésulfuration. A ce titre, plusieurs axes de recherche ont été développés. Parmi eux, les solutions techniques permettant de modifier les interactions support - précurseurs de phases oxydes sont prometteuses. Pour exemple, la demande de brevet WO 2007/084438A2 montre que l'emploi d'agents complexants, tels que l'acide nitrilotriacétique (ANT) ou l'acide éthylènediaminetétraacétique (EDTA), permet d'obtenir des catalyseurs d'hydrotraitement et notamment des catalyseurs d'hydrodésulfuration des essences supportés sur silice présentant une très bonne sélectivité vis à vis de la désulfuration des essences de craquage catalytique (FCC), en limitant la saturation des oléfines. Il a également été enseigné dans la demande de brevet EP-A-601.722 un catalyseur d'hydrotraitement d'huile préparé en présence d'un agent additif choisi parmi les dialcools ou les trialcools ayant de 2 à 10 atomes de carbone par molécule, les éthers desdits alcools, les monosaccharides ou disaccharides. L'ajout de ce type d'additifs permet d'améliorer l'activité des catalyseurs d'hydrotraitement.

### Résumé et intérêt de l'invention

La présente invention a pour objet un procédé d'hydrodésulfuration d'une coupe essence contenant des hydrocarbures ayant au moins 2 atomes de carbone par molécule et ayant un point d'ébullition final inférieur ou égal à 250°C, ledit procédé consistant en la mise en contact de ladite coupe essence avec au moins un catalyseur dont la phase active comprend au moins un métal du groupe VIII et au moins un métal du groupe VIB déposés sur un support formé d'au moins un oxyde, ledit catalyseur étant préparé selon un procédé comprenant au moins :
i) au moins une étape de mise en contact d'au moins dudit support avec au moins une solution contenant au moins un précurseur d'au moins dudit métal du groupe VIII et au moins un précurseur d'au moins dudit métal du groupe VIB,
ii) au moins une étape de mise en contact d'au moins dudit support avec au moins un composé organique formé d'au moins un oligosaccharide cyclique composé d'au moins 6 sous-unités glucopyranose liées en α-(1,4),
iii) au moins une étape de séchage pour obtenir au moins ledit métal du groupe VIII et au moins ledit métal du groupe VIB sous forme oxyde, puis
iv) au moins une étape de sulfuration de sorte que ladite phase active se présente sous forme sulfure,
les étapes i) et ii) pouvant être réalisées séparément, dans un ordre indifférent, ou simultanément.

Conformément au procédé d'hydrodésulfuration selon l'invention, ledit métal du groupe VIII présent dans la phase active est préférentiellement le cobalt et ledit métal du groupe VIB présent dans la phase active est préférentiellement le molybdène. Conformément au procédé d'hydrodésulfuration selon l'invention, ledit catalyseur est préférentiellement préparé en présence d'une cyclodextrine en tant que composé organique.

De façon surprenante, il a été découvert qu'un catalyseur sulfuré dont la phase active comprend au moins un métal du groupe VIII, préférentiellement un métal non-noble du groupe VIII, et au moins un métal du groupe VIB et préparé en présence d'au moins un composé organique formé d'au moins un oligosaccharide cyclique composé d'au moins 6 sous-unités glucopyranose liées en α-(1,4), préférentiellement d'une cyclodextrine, présente, lorsqu'il est mis en oeuvre dans un procédé d'hydrodésulfuration d'une coupe essence, des performances catalytiques améliorées, notamment en terme d'activité catalytique et/ou en terme de sélectivité. Il en résulte en particulier une réduction accentuée de la teneur en soufre de la coupe essence obtenue à l'issue dudit procédé d'hydrodésulfuration, notamment lorsque le support du catalyseur est à base d'alumine, et/ou l'obtention d'une coupe essence dont la perte d'indice d'octane est très faible, notamment lorsque le support est à base de silice.

### Description de l'invention

La présente invention a pour objet un procédé d'hydrodésulfuration d'une coupe essence contenant des hydrocarbures ayant au moins 2 atomes de carbone par molécule et ayant un point d'ébullition final inférieur ou égal à 250°C, ledit procédé consistant en la mise en contact de ladite coupe essence avec au moins un catalyseur dont la phase active comprend au moins un métal du groupe VIII et au moins un métal du groupe VIB déposés sur un support formé d'au moins un oxyde, ledit catalyseur étant préparé selon un procédé comprenant au moins :
i) au moins une étape de mise en contact d'au moins dudit support avec au moins une solution contenant au moins un précurseur d'au moins dudit métal du groupe VIII et au moins un précurseur d'au moins dudit métal du groupe VIB,
ii) au moins une étape de mise en contact d'au moins dudit support avec au moins un composé organique formé d'au moins un oligosaccharide cyclique composé d'au moins 6 sous-unités glucopyranose liées en α-(1,4),
iii) au moins une étape de séchage pour obtenir au moins ledit métal du groupe VIII et au moins ledit métal du groupe VIB sous forme oxyde, puis
iv) au moins une étape de sulfuration de sorte que ladite phase active se présente sous forme sulfure,
les étapes i) et ii) pouvant être réalisées séparément, dans un ordre indifférent, ou simultanément.

Ladite coupe essence, traitée dans le procédé d'hydrodésulfuration selon l'invention, est une coupe essence contenant du soufre et des hydrocarbures oléfiniques. Elle contient des hydrocarbures, en particulier des hydrocarbures oléfiniques, ayant au moins 2 atomes de carbone par molécule, préférentiellement au moins 5 atomes de carbone par molécule, et présente un point d'ébullition final inférieur ou égal à 250°C. Ladite coupe essence contient de manière préférée des hydrocarbures, en particulier des hydrocarbures oléfiniques, ayant de 5 à 8 atomes de carbone par molécule. Ladite coupe est préférentiellement issue d'une unité de cokéfaction (coking selon la terminologie anglo-saxonne), d'une unité de viscoréduction (visbreaking selon la terminologie anglo-saxonne), d'une unité de vapocraquage (steam cracking selon la terminologie anglo-saxonne) ou d'une unité de craquage catalytique en lit fluidisé (Fluid Catalytic Cracking, FCC selon la terminologie anglo-saxonne). De manière avantageuse, ladite coupe essence peut éventuellement être composée d'une fraction significative d'essence provenant d'autres procédés de production telle que la distillation atmosphérique (essence issue d'une distillation directe ou essence straight run selon la terminologie anglo-saxonne) ou de procédés de conversion (essence de cokéfaction ou de vapocraquage). Ladite coupe essence présente une teneur pondérale en soufre comprise entre 200 et 5000 ppm, de préférence entre 500 et 2000 ppm. Ladite coupe essence est très préférentiellement une coupe issue d'une unité de craquage catalytique en lit fluidisé. Une telle coupe essence issue d'une unité de craquage catalytique, avantageusement traitée dans le procédé d'hydrodésulfuration selon l'invention, contient environ 20 à 40 % poids de composés oléfiniques tels que le 2,3-diméthyl-1-butène, le 4,4-diméthylcyclopentène, le 2-méthyl-2-heptène, le 1-hexène, 30 à 60 % poids de composés aromatiques tels que l'éthylbenzène et forthoxylène, 20 à 50 % poids de composés saturés de type paraffines ou naphtènes tels que le 2-méthylhexane et le 1-méthylcyclopentane. Parmi les composés oléfiniques, les oléfines ramifiées, en particulier le 3-méthyl-cis-2-pentène et le 3-méthyl-cis-3-hexène, sont majoritaires par rapport aux oléfines linéaires et cycliques telles que le 1-hexène, le 1-heptène et le cyclopentène. Ladite coupe essence issue d'une unité de craquage catalytique, avantageusement traitée dans le procédé d'hydrodésulfuration selon l'invention, peut contenir une faible quantité, c'est-à-dire n'excédant pas 5% poids de la coupe essence, de composés polyinsaturés de type dioléfinique ou acétylénique, la présence de ces composés polyinsaturés étant préférentiellement comprise entre 100 ppm et 5% poids, très préférentiellement entre 100 ppm et 2% poids. Ladite coupe essence issue d'une unité de craquage catalytique présente préférentiellement une teneur pondérale en soufre comprise entre 200 et 5000 ppm, de préférence entre 500 et 2000 ppm.

Les composés soufrés présents dans la coupe essence à traiter selon le procédé d'hydrodésulfuration selon l'invention, en particulier présents dans la coupe essence issue d'une unité de craquage catalytique, sont notamment des composés thiophéniques tels que le 3-méthylthiophène et le 3,4-diméthylthiophène et des composés benzothiophéniques tel que le benzothiophène, les mercaptans (composés soufrés non cycliques présentant une liaison S-H), par exemple le propanethiol, n'étant présent qu'en faible quantité, c'est-à-dire en une teneur pondérale variant avantageusement entre 10 et 100 ppm.

Le procédé d'hydrodésulfuration selon l'invention vise à améliorer la conversion des composés soufrés présents dans la coupe essence par une activité catalytique accrue du catalyseur de manière à réduire la teneur en soufre dans la coupe essence destinée à être intégrée dans le pool essence et/ou à améliorer la sélectivité dudit catalyseur de manière à limiter l'hydrogénation des composés mono-oléfiniques et composés aromatiques ayant un indice d'octane élevé en composés saturés ayant un indice d'octane amoindri. Selon la composition du catalyseur mis en oeuvre, le procédé d'hydrodésulfuration selon l'invention permet une amélioration de l'activité catalytique sans dégradation de la sélectivité ou encore une amélioration de la sélectivité sans dégradation de l'activité catalytique.

La mise en oeuvre technologique du procédé d'hydrodésulfuration selon l'invention est par exemple réalisée par injection de la coupe essence et de l'hydrogène dans au moins un réacteur à lit fixe, à lit mobile ou lit bouillonnant, de manière préférée dans un réacteur à lit fixe.

Le procédé d'hydrodésulfuration selon l'invention est réalisé en phase gazeuse. Il est mis en oeuvre dans les conditions opératoires suivantes : une température comprise entre 200 et 400°C, préférentiellement entre 250 et 350°C, une pression totale comprise entre 1 et 3,5 MPa et plus préférentiellement entre 1 MPa et 2,5 MPa avec un rapport volume d'hydrogène sur volume de coupe essence compris entre 100 et 600 litres par litre et plus préférentiellement entre 200 et 400 litres par litre. Enfin, la vitesse volumique horaire (VVH) est l'inverse du temps de contact exprimée en heure. Elle est définie par le rapport du débit volumique de coupe essence liquide sur le volume de catalyseur chargé dans le réacteur. Elle est généralement comprise entre 1 et 10 h⁻¹, préférentiellement entre 2 et 8 h⁻¹.

Le catalyseur employé pour la mise en oeuvre du procédé d'hydrodésulfuration selon l'invention comprend une phase métallique active déposée sur un support, ladite phase active comprenant au moins un métal du groupe VIII de la classification périodique des éléments et au moins un métal du groupe VIB de la classification périodique des éléments. De manière préférée, la phase active dudit catalyseur comprend en outre du phosphore.

De manière générale, la teneur en métal(ux) du groupe VIB dans ledit catalyseur oxyde issu de ladite étape iii) est comprise entre 1 et 20 % poids d'oxyde(s) de métal(ux) du groupe VIB, de manière préférée entre 1,5 et 18 % poids d'oxyde(s) de métal(ux) du groupe VIB, de manière très préférée entre 2,5 et 18 % poids d'oxyde(s) de métal(ux) du groupe VIB. De préférence, le métal du groupe VIB est le molybdène ou le tungstène ou un mélange de ces deux éléments, et de manière plus préférée le métal du groupe VIB est constitué uniquement de molybdène ou de tungstène. Le métal du groupe VIB est de manière très préférée le molybdène.

De manière générale, la teneur en métal(ux) du groupe VIII dans ledit catalyseur oxyde issu de ladite étape iii) est comprise entre 0,1 et 20 % poids d'oxyde(s) de métal(ux) du groupe VIII, de manière préférée comprise entre 0,2 et 10 % poids d'oxyde(s) de métal(ux) du groupe VIII, de manière très préférée comprise entre 0,3 et 5 % poids d'oxyde(s) de métal(ux) du groupe VIII. De préférence, le métal du groupe VIII est un métal non noble du groupe VIII de la classification périodique des éléments. De manière très préférée, ledit métal du groupe VIII est le cobalt ou le nickel ou un mélange de ces deux éléments, et de manière plus préférée le métal du groupe VIII est constitué uniquement de cobalt ou de nickel. Le métal du groupe VIII est de manière très préférée le cobalt.

Le rapport molaire métal(ux) du groupe VIII sur métal(ux) du groupe VIB dans le catalyseur oxyde issu de ladite étape iii) est préférentiellement compris entre 0,1 et 0,8, très préférentiellement compris entre 0,2 et 0,6, et de manière encore plus préférée compris entre 0,3 et 0,5.

Lorsque le catalyseur contient du phosphore, la teneur en phosphore dans ledit catalyseur oxyde issu de ladite étape iii) est de préférence comprise entre 0,1 et 10 % poids de P₂O₅, de manière plus préférée entre 0,2 et 5 % poids de P₂O₅, de manière très préférée entre 0,3 et 4 % poids de P₂O₅, de manière encore plus préférée entre 0,35 et 3 % poids de P₂O₅.

Le rapport molaire phosphore sur métal(ux) du groupe VIB dans le catalyseur oxyde issu de ladite étape iii) est supérieur ou égal à 0,25, de préférence supérieur ou égal à 0,27, de manière plus préférée compris entre 0,27 et 2,00, de manière encore plus préférée compris entre 0,35 et 1,40.

Le support sur lequel est déposée la phase active est avantageusement formé d'au moins un solide poreux sous forme oxyde choisi dans le groupe constitué par les alumines, les silices, les silices-alumine ou encore les oxydes de titane ou de magnésium utilisé(s) seul ou en mélange avec l'alumine ou la silice-alumine. Il est de préférence choisi dans le groupe constitué par les silices, les alumines de transition et les silices-alumine. De manière très préférée, le support est essentiellement constitué d'une alumine de transition ou de la silice. Un support essentiellement constitué d'une alumine de transition comprend au moins 51 % poids, de préférence au moins 60 % poids, de manière très préféré au moins 80 % poids, voire au moins 90 % poids de ladite alumine de transition. Par alumine de transition, on entend par exemple une alumine phase alpha, une alumine phase delta, une alumine phase gamma ou un mélange d'alumine de ces différentes phases. Un support essentiellement constitué de silice comprend au moins 51 % poids, de préférence au moins 60 % poids, de manière très préféré au moins 80 % poids, voire au moins 90 % poids de silice. Selon un premier mode très préféré, ledit support est constitué uniquement d'une alumine de transition. Selon un deuxième mode très préféré, ledit support est constitué uniquement de silice. Le volume poreux du support est généralement compris entre 0,4 et 1,4 cm³/g, de préférence compris entre 0,5 et 1,3 cm³/g. La surface spécifique du support est généralement comprise entre 40 et 300 m²/g, de préférence entre 60 et 250 m²/g. Ledit support poreux se présente avantageusement sous forme de billes, d'extrudés, de pastilles, ou d'agglomérats irréguliers et non sphériques dont la forme spécifique peut résulter d'une étape de concassage. De manière très avantageuse, ledit support se présente sous forme de billes ou d'extrudés.

Le catalyseur mis en oeuvre dans le procédé d'hydrodésulfuration selon l'invention est préparé selon un procédé comprenant au moins :
i) au moins une étape de mise en contact d'au moins dudit support avec au moins une solution contenant au moins un précurseur d'au moins dudit métal du groupe VIII et au moins un précurseur d'au moins dudit métal du groupe VIB,
ii) au moins une étape de mise en contact d'au moins dudit support avec au moins un composé organique formé d'au moins un oligosaccharide cyclique composé d'au moins 6 sous-unités glucopyranose liées en α-(1,4),
iii) au moins une étape de séchage pour obtenir au moins ledit métal du groupe VIII et au moins ledit métal du groupe VIB sous forme oxyde, puis,
iv) au moins une étape de sulfuration de sorte que ladite phase active se présente sous forme sulfure,
les étapes i) et ii) pouvant être réalisées séparément, dans un ordre indifférent, ou simultanément.

Le dépôt d'au moins dudit métal du groupe VIII et d'au moins dudit métal du groupe VIB sur ledit support, conformément à la mise en oeuvre de ladite étape i), peut être réalisé par toute méthode bien connue de l'Homme du métier. Ladite étape i) est préférentiellement réalisée par imprégnation du support par au moins une solution contenant au moins un précurseur dudit métal du groupe VIII et au moins un précurseur dudit métal du groupe VIB. En particulier, ladite étape i) peut être réalisée par imprégnation à sec, par imprégnation en excès, ou encore par dépôt - précipitation selon des méthodes bien connues de l'Homme du métier. De manière préférée, ladite étape i) est réalisée par imprégnation à sec, laquelle consiste à mettre en contact le support du catalyseur avec au moins une solution, contenant au moins un précurseur dudit métal du groupe VIII et au moins un précurseur dudit métal du groupe VIB, dont le volume est égal au volume poreux du support à imprégner. Cette solution contient les précurseurs métalliques du ou des métaux du groupe VIII et du ou des métaux du groupe VIB à la concentration voulue. Ladite solution peut également contenir de l'eau oxygénée, notamment dans le cas où le métal du groupe VIII est le cobalt et le métal du groupe VIB est le molybdène.

Le(s)dit(s) métal(ux) du groupe VIII et le(s)dit(s) métal(ux) du groupe VIB sont mis au contact dudit support par l'intermédiaire de tout précurseur métallique soluble en phase aqueuse ou en phase organique. De manière préférée, le(s)dit(s) précurseur(s) du(es) métal(ux) du groupe VIII et le(s)dit(s) précurseur(s) du(es) métal(ux) du groupe VIB sont introduits en solution aqueuse. Lorsque le métal du groupe VIII est le cobalt, on utilise avantageusement comme précurseur le nitrate de cobalt, l'hydroxyde de cobalt ou le carbonate de cobalt. Lorsque le métal du groupe VIII est le nickel, on utilise avantageusement comme précurseur le nitrate de nickel, l'hydroxyde de nickel ou le carbonate de nickel. Lorsque ledit métal du groupe VIB est le molybdène, on utilise avantageusement l'heptamolybdate d'ammonium ou l'oxyde de molybdène. Lorsque ledit métal du groupe VIB est le tungstène, on utilise avantageusement le métatungstate d'ammonium. Lorsque le phosphore est présent dans la phase active du catalyseur, on utilise avantageusement comme précurseur l'acide phosphorique. Tout autre sel connu de l'homme du métier présentant une solubilité suffisante en solution aqueuse et décomposable lors d'une étape de séchage, en particulier lors de l'étape de séchage selon ladite étape iii), peut également être utilisé.

La mise en contact dudit composé organique employé pour la mise en oeuvre de ladite étape ii) avec ledit support est réalisée par imprégnation, notamment par imprégnation à sec ou imprégnation en excès, préférentiellement par imprégnation à sec. Ledit composé organique est préférentiellement imprégné sur ledit support après solubilisation en solution aqueuse. La solution d'imprégnation comprend avantageusement un acide, par exemple de l'acide acétique.

Ledit composé organique est formé d'au moins un oligosaccharide cyclique composé d'au moins 6 sous-unités glucopyranose liées en α-(1,4). Une représentation spatiale d'une sous-unité glucopyranose est donnée ci-dessous :

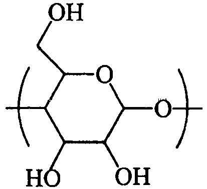

Ledit composé organique est préférentiellement choisi parmi les cyclodextrines, les cyclodextrines substituées, les cyclodextrines polymérisées et les mélanges de cyclodextrines. Les cyclodextrines sont une famille d'oligosaccharides cycliques composés de sous-unités glucopyranose liées en α-(1,4). Il s'agit de molécules-cages. Selon l'invention, les cyclodextrines préférées sont l'a-cyclodextrine, la β-cyclodextrine et la γ-cyclodextrine respectivement composée de 6, 7 et 8 sous-unités glucopyranose liées en α-(1,4). Les représentations développées de l'α-cyclodextrine, de la β-cyclodextrine et de la γ-cyclodextrine sont données ci-dessous. On utilise préférentiellement pour la mise en oeuvre de ladite étape ii) la β-cyclodextrine composée de 7 sous-unités glucopyranose liées en α-(1,4). Les cyclodextrines sont des composés commercialisés.

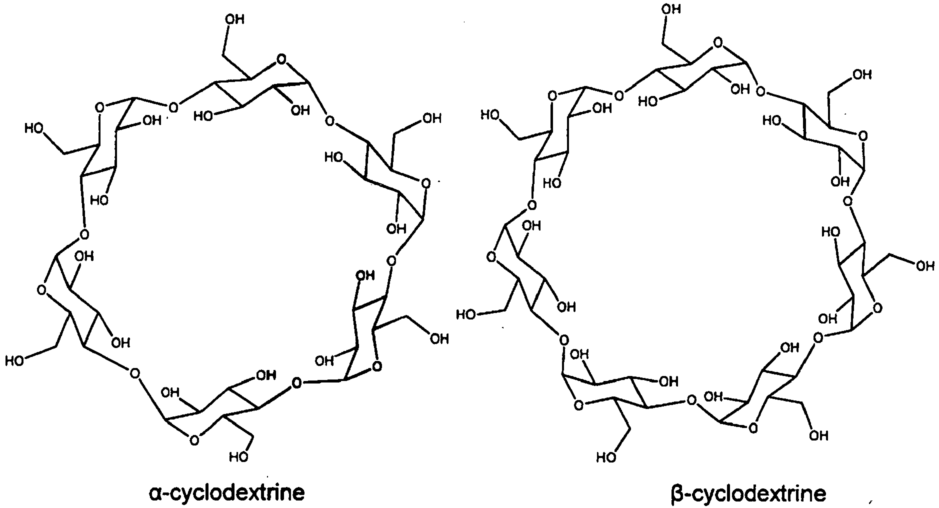

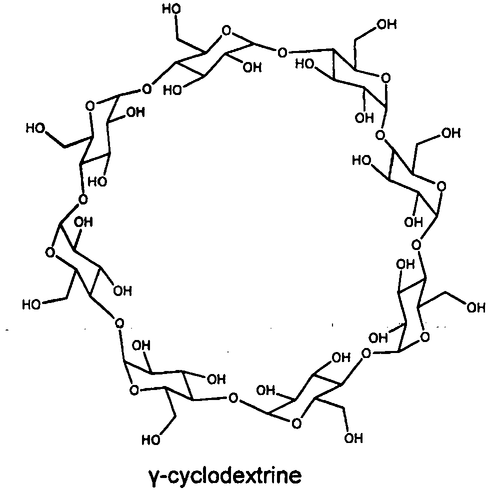

Les cyclodextrines substituées avantageusement employées pour la mise en oeuvre de ladite étape ii) sont constituées de 6, 7 ou 8 sous-unités glucopyranose liées en α-(1,4), dont l'une au moins est mono- ou polysubstituée. Les substituants peuvent être fixés sur un ou plusieurs groupe(s) hydroxyle(s) présent(s) dans la molécule, à savoir sur les groupes hydroxyles directement liés au cycle d'une unité glucopyranose et/ou sur l'hydroxyle lié au groupement CH₂ lui-même lié au cycle d'une unité glucopyranose. De manière plus préférée, lesdites cyclodextrines substituées portent un ou plusieurs substituant(s), identique(s) ou différent(s), choisi(s) parmi les radicaux alkyles saturés ou non, fonctionnalisés ou non, et les fonctions esters, carbonyles, carboxyles, carboxylates, phosphates, éthers, polyéthers, urées, amide, amino, triazole, ammonium. Les cyclodextrines substituées préférées sont les cyclodextrines méthylées, éthylées, propylées, allylées (c'est-à-dire possédant une fonction ayant pour formule semi-développée -CH₂-CH=CH₂), succinylées (c'est-à-dire possédant une fonction ayant pour formule semi-développée R-OCO-CH₂-CH₂COOH), carboxylées, carboxyméthylées, acétylées, 2-hydroxypropylées et polyoxyéthylénées. Les groupements mono- ou polysubstituants de la cyclodextrine peuvent aussi être une molécule de monosaccharide ou disaccharide telle qu'une molécule de maltose, glucose, fructose ou saccharose.

Les cyclodextrines substituées particulièrement avantageuses pour la mise en oeuvre de ladite étape ii) sont l'hydroxypropyl bêta-cyclodextrine et les bêta-cyclodextrines méthylées.

Les cyclodextrines polymérisées avantageusement employées pour la mise en oeuvre de ladite étape ii) sont des polymères dont les monomères sont chacun constitué d'un oligosaccharide cyclique composé de 6, 7 ou 8 sous-unités glucopyranose liées en α-(1,4), substituées ou non. Une cyclodextrine sous forme polymérisée, réticulée ou non, pouvant être avantageusement utilisée pour la mise en oeuvre de ladite étape ii) est par exemple du type de celle obtenue par polymérisation de monomères de bêta-cyclodextrine avec de l'épichlorhydrine ou un polyacide.

Les mélanges de cyclodextrines avantageusement employés pour la mise en oeuvre de ladite étape ii) mettent en oeuvre des cyclodextrines substituées ou non. Lesdits mélanges pourront, à titre d'exemple, contenir conjointement et dans des proportions variables, chacun des trois types de cyclodextrines (alpha, bêta et gamma).

L'introduction dudit composé organique, préférentiellement d'une cyclodextrine et très préférentiellement de la bêta-cyclodextrine, pour la mise en oeuvre de ladite étape ii) est telle que le rapport molaire {(métal(ux) des groupes (VIII + VIB) sous forme d'oxyde présent dans phase active du catalyseur obtenu à l'issue de ladite étape iii)/composé organique} est compris entre 10 et 300 et de préférence entre 25 et 180. Les métaux des groupes VIII et VIB pris en compte pour le calcul dudit rapport molaire sont les métaux introduits pour la mise en oeuvre de ladite étape i) et se trouvant sous la forme d'oxyde dans la phase active du catalyseur issu de ladite étape iii). Lesdits métaux du groupe VIII et VIB peuvent en conséquence se trouver sous la forme sulfure : ils seront sulfurés préalablement à la mise en oeuvre du procédé d'hydrodésulfuration selon l'invention.

Le procédé de préparation du catalyseur utilisé dans le procédé d'hydrodésulfuration selon l'invention comporte plusieurs modes de mises en oeuvre.

Un premier mode de mise en oeuvre consiste à effectuer lesdites étapes i) et ii) de façon simultanée de sorte que ledit composé organique, de préférence une cyclodextrine, et au moins ledit précurseur d'au moins dudit métal du groupe VIII et au moins ledit précurseur d'au moins dudit métal du groupe VIB présents dans la phase active sont co-imprégnés sur ledit support (étape de co-imprégnation). Ledit premier mode de mise en oeuvre comprend avantageusement la mise en oeuvre d'une ou plusieurs étapes i). En particulier, une ou plusieurs étapes i) précède(nt) et/ou suive(nt) avantageusement ladite étape de co-imprégnation. Conformément audit premier mode de mise en oeuvre, chacune des étapes de (co)-imprégnation réalisées est préférentiellement suivie d'une étape de maturation puis d'au moins une étape de séchage puis éventuellement d'au moins une étape de calcination, ladite étape de calcination étant préférentiellement réalisée lorsque le support comprend, de préférence est constitué, de l'alumine. En particulier, ladite étape de co-imprégnation est suivie d'au moins une étape de séchage conformément à ladite étape iii). Ledit premier mode de mise en oeuvre peut comprendre plusieurs étapes de co-imprégnation.

Un deuxième mode de mise en oeuvre consiste à effectuer ladite étape i) préalablement à ladite étape ii). Conformément audit deuxième mode de mise en oeuvre, une ou plusieurs étapes i) de dépôt d'au moins dudit métal du groupe VIII et d'au moins dudit métal du groupe VIB présents dans la phase active du catalyseur précède(nt) ladite étape ii). De préférence, chacune desdites étapes i) est immédiatement suivie d'une étape de maturation puis d'au moins une étape de séchage. En particulier, la dernière étape i) est avantageusement suivie d'au moins une étape de séchage conformément à ladite étape iii) avant la mise en oeuvre de ladite étape ii). Ladite étape ii) est avantageusement suivie d'une étape de maturation puis d'au moins une étape de séchage, laquelle est réalisée dans les mêmes conditions que celles opérées pour ladite étape iii), et éventuellement d'au moins une étape de calcination, ladite étape de calcination étant préférentiellement réalisée lorsque le support comprend, de préférence est constitué, de l'alumine.

Un troisième mode de mise en oeuvre consiste à effectuer ladite étape ii) préalablement à ladite étape i). Ladite étape ii) est préférentiellement immédiatement suivie d'une étape de maturation puis d'au moins une étape de séchage et éventuellement d'au moins une étape de calcination avant la mise en oeuvre de ladite étape i). De manière avantageuse, ladite étape ii) est suivie de plusieurs étapes i). La préparation du catalyseur conformément audit troisième mode de réalisation se termine avantageusement par ladite étape iii) de séchage, laquelle est éventuellement suivie d'une étape de calcination.

Chacun des trois modes de mises en oeuvre décrits ci-dessus peut être effectué de manière indépendante de sorte que le catalyseur utilisé dans le procédé selon l'invention est préparé soit selon ledit premier mode de mise en oeuvre, soit selon ledit deuxième mode de mise en oeuvre soit encore selon ledit troisième mode de mise en oeuvre. Toutefois, il peut être avantageux d'associer ledit premier mode avec ledit deuxième mode ou avec ledit troisième mode : aussi bien les métaux du groupe VIII et du groupe VIB présents dans la phase active que le composé organique, préférentiellement une cyclodextrine, sont déposés au moins à deux reprises sur le support du catalyseur, à savoir au moins une fois par co-imprégnation et au moins une fois par imprégnation successive.

Ladite étape iii) de séchage, mise en oeuvre pour la préparation du catalyseur, préparé selon au moins un mode de mise en oeuvre décrit ci-dessus, est réalisée à une température comprise entre 80 et 160°C. Elle est préférentiellement réalisée pendant une durée comprise entre 1 et 20 heures. Ladite étape iii) est avantageusement suivie d'au moins une étape de calcination, en particulier lorsque le support comprend, de préférence est constitué, de l'alumine. L'étape éventuelle de calcination est réalisée à une température comprise entre 200 et 550°C, de préférence entre 300 et 500°C. Elle est préférentiellement réalisée pendant une durée comprise entre 1 et 6 heures.

Le catalyseur, obtenu à l'issue de ladite étape iii), après mise en oeuvre des étapes i) et ii) selon au moins l'un des trois modes de mises en oeuvre décrits ci-dessus, se trouve à l'état oxyde.

La préparation du catalyseur, utilisé dans le procédé d'hydrodésulfuration d'une coupe essence selon l'invention, comprend au moins une étape iv) de sulfuration de sorte que ladite phase active se présente sous forme sulfure. Ladite étape iv) est effectuée après la mise en oeuvre des étapes i), ii) et iii). Elle est réalisée par la mise en contact dudit catalyseur, obtenu à l'issue de la mise en oeuvre desdites étapes i), ii) et iii) et éventuellement d'une étape de calcination subséquente, avec au moins un composé organique soufré décomposable et générateur d'H₂S ou par la mise en contact direct dudit catalyseur avec un flux gazeux d'H₂S dilué dans l'hydrogène. Ladite étape iv) de sulfuration peut être réalisée *in situ* (c'est-à-dire après chargement du catalyseur dans l'unité réactionnelle du procédé d'hydrodésulfuration selon l'invention) ou *ex situ* (c'est-à-dire avant chargement du catalyseur dans l'unité réactionnelle du procédé d'hydrodésulfuration selon l'invention) à une température comprise entre 200 et 600°C et plus préférentiellement entre 300 et 500°C.

Le catalyseur, issu de ladite étape iv), se trouve avant la mise en oeuvre du procédé d'hydrodésulfuration selon l'invention, au moins partiellement sous forme sulfurée. Il peut également comprendre une phase métallique oxyde, qui n'a pas été transformée lors de ladite étape de sulfuration iv). Ledit catalyseur peut être entièrement ou partiellement débarrassé dudit composé organique formé d'au moins un oligosaccharide cyclique composé d'au moins 6 sous-unités glucopyranose liées en α-(1,4).

L'invention est illustrée par les exemples qui suivent.

### EXEMPLES

Les catalyseurs A1, A2, A3 et A4 préparés respectivement dans les exemples 1, 2, 3 et 4 sont préparés à isoteneur en éléments molybdène, cobalt et phosphore. Le support utilisé pour la préparation de chacun des catalyseurs A1, A2, A3 et A4 est un support de billes d'alumine, ayant un volume poreux de 1,08 ml/g et une surface BET égale à 81 m²/g.

Les catalyseurs B1, B2 et B3 préparés respectivement dans les exemples 5, 6 et 7 sont préparés à isoteneur en éléments molybdène et cobalt. Le support utilisé pour la préparation des catalyseurs B1, B2 et B3 est un support d'extrudés de silice ayant un volume poreux de 0,99 ml/g et une surface BET égale à 238 m²/g.

### Exemple 1 (comparatif) : Préparation d'un catalyseur supporté A1 (catalyseur oxyde) et d'un catalyseur supporté A1' (catalyseur sulfure) de formule CoMoP/Al₂O₃

Le catalyseur A1 est obtenu par imprégnation à sec d'une solution aqueuse préparée à partir d'oxyde de molybdène, d'hydroxyde de cobalt et d'acide phosphorique, le volume de ladite solution contenant les précurseurs de cobalt, de molybdène et de phosphore étant rigoureusement égal au volume poreux de la masse de support d'alumine. Les concentrations en précurseurs dans la solution aqueuse sont ajustées de manière à déposer sur le support d'alumine les teneurs pondérales en Co, Mo et P souhaitées. Après une étape de maturation pendant 12 heures, le solide est séché pendant 12 heures à 120°C. Le solide est ensuite calciné sous air à 450°C pendant 2 heures pour obtenir le catalyseur A1.

Le catalyseur A1 ainsi obtenu à l'état oxyde, de formulation CoMoP, présente une teneur en molybdène de 7,6 exprimé en % poids d'oxyde MoO₃, une teneur en cobalt de 1,4 exprimé en % poids d'oxyde CoO et une teneur en phosphore de 1,5 exprimé en % poids d'oxyde P₂O₅. Le rapport molaire Co/Mo de ce catalyseur est de 0,35 et le ratio molaire P/Mo de 0,40.

Le catalyseur A1 est sulfuré *ex situ* en phase gazeuse à 500°C pendant 2 heures sous flux d'H₂S dans l'hydrogène (15% vol de H₂S dans H₂). On obtient un catalyseur A1' sous forme sulfure.

### Exemple 2 (invention) : Préparation d'un catalyseur supporté A2 (catalyseur oxyde) et d'un catalyseur supporté A2' (catalyseur sulfure) de formule CoMoP/Al₂O₃ en Présence de β-cyclodextrine (co-imprégnation)

Le catalyseur A2 est obtenu par imprégnation à sec d'une solution aqueuse préparée à partir d'oxyde de molybdène, d'hydroxyde de cobalt et d'acide phosphorique, le volume de ladite solution contenant les précurseurs de cobalt, de molybdène et de phosphore étant rigoureusement égal au volume poreux de la masse de support d'alumine. Les concentrations en précurseurs dans la solution aqueuse sont ajustées de manière à déposer sur le support d'alumine les teneurs pondérales en Co, Mo et P souhaitées. Ladite solution aqueuse contient également de la β-cyclodextrine (SIGMA-ALDRICH, pureté de 98%), dans un rapport molaire (Co+Mo)/β-cyclodextrine de 30. Après une étape de maturation de 12 heures, le solide est séché pendant 12 heures à 120°C. Le solide est ensuite calciné sous air à 450°C pendant 2 heures pour obtenir le catalyseur A2.

Le catalyseur A2 ainsi obtenu à l'état oxyde, de formulation CoMoP, présente une teneur en molybdène de 7,5 exprimé en % poids d'oxyde MoO₃, une teneur en cobalt de 1,5 exprimé en % poids d'oxyde CoO et une teneur en phosphore de 1,5 exprimé en % poids d'oxyde P₂O₅. Le rapport molaire Co/Mo de ce catalyseur est de 0,38 et le ratio molaire P/Mo de 0,38.

Le catalyseur A2 est sulfuré *ex situ* en phase gazeuse à 500°C pendant 2 heures sous flux d'H₂S dans l'hydrogène (15% vol de H₂S dans H₂). On obtient un catalyseur A2' sous forme sulfure.

### Exemple 3 (invention): Préparation d'un catalyseur supporté A3 (catalyseur oxyde) et d'un catalyseur supporté A3' (catalyseur sulfure) de formule CoMoP/Al₂O₃ en présence de β-cyclodextrine (co-imprégnation de Mo, Co, P puis imprégnation successive de β-cyclodextrine)

Le catalyseur A3 est obtenu par imprégnation à sec d'une solution aqueuse préparée à partir d'oxyde de molybdène, d'hydroxyde de cobalt et d'acide phosphorique, le volume de ladite solution contenant les précurseurs de cobalt, de molybdène et de phosphore étant rigoureusement égal au volume poreux de la masse de support d'alumine. Les concentrations en précurseurs dans la solution aqueuse sont ajustées de manière à déposer sur le support d'alumine les teneurs pondérales en Co, Mo et P souhaitées. Après une étape de maturation de 12 heures, le solide est séché pendant 12 heures à 120°C. Une deuxième étape d'imprégnation à sec permet d'ajouter la β-cyclodextrine (SIGMA-ALDRICH, pureté de 98%) dissoute dans l'eau au solide séché obtenu préalablement. Le rapport molaire (Co+Mo)/β-cyclodextrine est de 30. Après une étape de maturation 12 heures, le solide est séché pendant 12 heures à 120°C. Le solide est ensuite calciné sous air à 450°C pendant 2 heures pour obtenir le catalyseur A3.

Le catalyseur A3 ainsi obtenu à l'état oxyde, de formulation CoMoP, a une teneur en molybdène de 7,5 exprimé en % poids d'oxyde MoO₃, une teneur en cobalt de 1,4 exprimé en % poids d'oxyde CoO et une teneur en phosphore de 1,4 exprimé en % poids d'oxyde P₂O₅. Le rapport molaire Co/Mo de ce catalyseur est de 0,36 et le ratio molaire P/Mo de 0,41.

Le catalyseur A3 est sulfuré *ex situ* en phase gazeuse à 500°C pendant 2 heures sous flux d'H₂S dans l'hydrogène (15% vol de H₂S dans H₂). On obtient un catalyseur A3' sous forme sulfure.

### Exemple 4 (comparatif): Préparation d'un catalyseur supporté A4 (catalyseur oxyde) et d'un catalyseur supporté A4' (catalyseur sulfure) de formule CoMoP/Al₂O₃ en présence de cellobiose (co-imprégnation)

Le cellobiose ou β-D-glucopyrannosyl(1→4)D-glucopyrannose est le produit de la dégradation de la cellulose. Il s'agit d'un diholoside de formule brute C₁₂H₂₂O₁₁. Il ne s'agit pas d'un oligosaccharide cyclique. La formule développée du cellobiose est donnée ci-dessous :

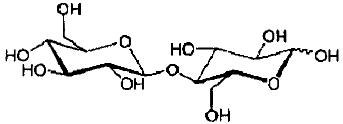

Le catalyseur A4 est obtenu par imprégnation à sec d'une solution aqueuse préparée à partir d'oxyde de molybdène, d'hydroxyde de cobalt et d'acide phosphorique, le volume de ladite solution contenant les précurseurs de cobalt, de molybdène et de phosphore étant rigoureusement égal au volume poreux de la masse de support. Les concentrations en précurseurs dans la solution aqueuse sont ajustées de manière à déposer sur le support les teneurs pondérales en Co, Mo et P souhaitées. La solution aqueuse contient également du cellobiose (commercialisée par VWR), dans un rapport molaire (Co+Mo)/cellobiose de 30. Après une étape de maturation de 12 heures, le solide est séché pendant 12 heures à 120°C. Le solide est ensuite calciné sous air à 450°C pendant 2 heures pour obtenir le catalyseur A4.

Le catalyseur A4 ainsi obtenu à l'état oxyde, de formulation CoMoP, présente une teneur en molybdène de 7,4 exprimé en % poids d'oxyde MoO₃, une teneur en cobalt de 1,5 exprimé en % poids d'oxyde CoO et une teneur en phosphore de 1,5 exprimé en % poids d'oxyde P₂O₅. Le rapport molaire Co/Mo de ce catalyseur est de 0,39 et le ratio molaire P/Mo de 0,41.

Le catalyseur A4 est sulfuré ex *situ* en phase gazeuse à 500°C pendant 2 heures sous flux d'H₂S dans l'hydrogène (15% vol de H₂S dans H₂). On obtient un catalyseur A4' sous forme sulfure.

### Exemple 5 (comparatif) : Préparation d'un catalyseur supporté B1 (catalyseur oxyde) et d'un catalyseur B1' (catalyseur sulfure) de formule CoMo/SiO₂

Le catalyseur B1 est obtenu par imprégnation à sec d'une solution aqueuse préparée à partir d'oxyde de molybdène, de carbonate de cobalt et d'eau oxygénée, avec un rapport molaire H₂O₂/MoO₃ de 4,5, le volume de la solution aqueuse contenant les précurseurs des métaux étant rigoureusement égal au volume poreux de la masse de support de silice. Les concentrations en précurseurs de Mo et Co dans la solution aqueuse sont ajustées de manière à déposer sur le support de silice les teneurs pondérales en Co et Mo souhaitées. Après une étape de maturation de 12 heures, le solide est ensuite séché pendant 12 heures à 120°C.

Le catalyseur B1 ainsi obtenu à l'état oxyde, de formulation CoMo a une teneur en molybdène de 17,8 exprimé en % poids d'oxyde MoO₃, et une teneur en cobalt de 4,6 exprimé en % poids d'oxyde CoO. Le rapport molaire Co/Mo de ce catalyseur est de 0,50.

Le catalyseur B1 est sulfuré *ex situ* en phase gazeuse à 500°C pendant 2 heures sous flux d'H₂S dans l'hydrogène (15% vol de H₂S dans H₂). On obtient un catalyseur B1' sous forme sulfure.

### Exemple 6 (invention) : Préparation d'un catalyseur supporté B2 (catalyseur oxyde) et d'un catalyseur supporté B2' (catalyseur sulfure) de formule CoMo/SiO₂ en présence de β-cyclodextrine (co-imprégnation)

Le catalyseur est obtenu par imprégnation à sec d'une solution aqueuse préparée à partir d'oxyde de molybdène, de carbonate de cobalt et d'eau oxygénée, avec un rapport molaire H₂O₂/MoO₃ de 4,5, le volume de ladite solution aqueuse contenant les précurseurs des métaux étant rigoureusement égal au volume poreux de la masse de support de silice. Les concentrations en précurseurs de Mo et Co dans la solution aqueuse sont ajustées de manière à déposer sur le support de silice les teneurs pondérales en Co et Mo souhaitées. La solution aqueuse contient également de la β-cyclodextrine (SIGMA-ALDRICH, pureté de 98%), dans un rapport molaire (Co+Mo)/β-cyclodextrine de 30. Après une étape de maturation de 12 heures, le solide est ensuite séché pendant 12 heures à 120°C.

Le catalyseur B2 ainsi obtenu à l'état oxyde, de formulation CoMo présente une teneur en molybdène de 17,7 exprimé en % poids d'oxyde MoO₃, et une teneur en cobalt de 4,6 exprimé en % poids d'oxyde CoO. Le rapport molaire Co/Mo de ce catalyseur est de 0,50. Le catalyseur B2 est sulfuré *ex situ* en phase gazeuse à 500°C pendant 2 heures sous flux d'H₂S dans l'hydrogène (15% vol de H₂S dans H₂). On obtient un catalyseur B2' sous forme sulfure.

### Exemple 7 (comparatif) : Préparation d'un catalyseur supporté B3 (catalyseur oxyde) et d'un catalyseur supporté B3' (catalyseur sulfure) de formule CoMo/SiO₂ en présence de cellobiose (co-imprégnation)

Le catalyseur est obtenu par imprégnation à sec d'une solution aqueuse préparée à partir d'oxyde de molybdène, de carbonate de cobalt et d'eau oxygénée, avec un rapport molaire H₂O₂/MoO₃ de 4,5, le volume de ladite solution aqueuse contenant les précurseurs des métaux étant rigoureusement égal au volume poreux de la masse de support de silice. Les concentrations en précurseurs de Co et Mo dans la solution aqueuse sont ajustées de manière à déposer sur le support de silice les teneurs pondérales en Co et Mo souhaitées. La solution aqueuse contient également du cellobiose (commercialisée par VWR), dans un rapport molaire (Co+Mo)/cellobiose de 30. Après une étape de maturation de 12 heures, le solide est ensuite séché pendant 12 heures à 120°C.

Le catalyseur B3 ainsi obtenu à l'état oxyde, de formulation CoMo présente une teneur en molybdène de 17,9 exprimé en % poids d'oxyde MoO₃, et une teneur en cobalt de 4,7 exprimé en % poids d'oxyde CoO. Le rapport molaire Co/Mo de ce catalyseur est de 0,50. Le catalyseur B3 est sulfuré *ex situ* en phase gazeuse à 500°C pendant 2 heures sous flux d'H₂S dans l'hydrogène (15% vol de H₂S dans H₂). On obtient un catalyseur B3' sous forme sulfure.

### Exemple 8 : Performances catalytiques des catalyseurs A1', A2', A3', A4', B1', B2' et B3' en test d'hydrodésulfuration d'une coupe essence à partir de molécules modèles représentatives d'une essence de craquage catalytique

Une charge modèle représentative d'une essence de craquage catalytique (FCC) contenant 10 % poids de 2,3-diméthylbut-2-ène et 0,33 % poids de 3-méthylthiophène (soit 1000 ppm de soufre dans la charge) est utilisée pour l'évaluation des performances catalytiques des différents catalyseurs. Le solvant utilisé est l'heptane.

La réaction d'hydrodésulfuration est opérée dans un réacteur fermé de type Grignard sous une pression totale de 3,5 MPa, à 250°C. Chacun des catalyseurs A1', A2', A3', A4', B1', B2' et B3' sont placés successivement dans ledit réacteur. Des échantillons sont prélevés à différents intervalles de temps et sont analysés par chromatographie en phase gazeuse de façon à observer la disparition des réactifs.

Les performances catalytiques des catalyseurs A1', A2', A3', A4', B1', B2' et B3' sont évaluées à partir de l'activité catalytique et de la sélectivité.

L'activité du catalyseur est exprimée en constante de vitesse kHDS de la réaction d'hydrodésulfuration (HDS), normalisée par volume de catalyseur sous forme sulfure, en supposant un ordre 1 par rapport aux composés soufrés. La sélectivité du catalyseur est exprimée en rapport normalisé des constantes de vitesse kHDS/kHDO, kHDO étant la constante de vitesse pour la réaction d'hydrogénation des oléfines (HDO), à savoir dans le cas présent pour la réaction d'hydrogénation du 2,3-diméthylbut-2-ène, normalisée par volume de catalyseur sous forme sulfure, en supposant un ordre 1 par rapport aux oléfines. Le rapport kHDS/kHDO sera d'autant plus élevé que le catalyseur sera plus sélectif, signifiant une hydrogénation limitée du 2,3-diméthylbut-2-ène. Une augmentation du rapport kHDS/kHDO est donc favorable sur la qualité de l'essence obtenue à l'issue de la réaction d'hydrodésulfuration, dès lors que l'hydrogénation des oléfines ayant été limitée, la perte d'indice d'octane de l'essence résultante est fortement minimisée.

Les performances des catalyseurs supportés sur alumine sont données dans le tableau 1. Les valeurs sont normalisées en prenant le catalyseur A1' comme référence et en prenant kHDS/kHDO=100 et kHDS=100.

**Tableau 1 : Performances sur charge modèle des catalyseurs supportés sur alumine**

| Catalyseur | k HDS | kHDS/kHDO |
|---|---|---|
| A1' (comparatif) | 100 | 100 |
| A2' (invention) | 131 | 101 |
| A3' (invention) | 139 | 102 |
| A4' (comparatif) | 105 | 99 |

Les performances des catalyseurs supportés sur silice sont données dans le tableau 2. Les valeurs sont normalisées en prenant le catalyseur B1' comme référence et en prenant kHDS/kHDO=100 et kHDS=100.

**Tableau 2 : Performances sur charge modèle des catalyseurs supportés sur silice**

| Catalyseur | k HDS | kHDS/kHDO |
|---|---|---|
| B1' (comparatif) | 100 | 100 |
| B2' (invention) | 102 | 135 |
| B3' (comparatif) | 101 | 104 |

Les résultats figurant dans le tableau 1 démontrent que sur alumine, l'ajout de β-cyclodextrine (catalyseurs A2' et A3') permet un gain significatif de l'activité catalytique sans modification de la sélectivité par rapport au catalyseur A1' préparé en l'absence de β-cyclodextrine. Aucune amélioration sensible des performances catalytiques, ni en terme d'activité catalytique ni en terme de sélectivité, n'est observée lorsque le catalyseur supporté sur alumine est préparé en présence de cellobiose (catalyseur A4') qui n'appartient pas à la famille des oligosaccharides cycliques. Les catalyseurs A2' et A3' sont plus actifs que les catalyseurs A1' et A4'. Il en résulte une meilleure élimination du soufre dans des conditions opératoires identiques.

Les résultats figurant dans le tableau 2 démontrent que sur silice, l'ajout de β-cyclodextrine (catalyseur B2') permet un gain significatif de la sélectivité sans détérioration de l'activité par rapport au catalyseur B1' préparé en l'absence de β-cyclodextrine. L'ajout de cellobiose (catalyseur B3') ne permet pas d'améliorer nettement les performances catalytiques. Le catalyseur B2' est donc plus sélectif que les catalyseurs B1' et B3' : il limite l'hydrogénation de 2,3-diméthylbut-2-ène en 2,3-diméthylbut-2-ane permettant l'obtention d'une essence de meilleure qualité (meilleur indice d'octane) que celle obtenue avec les catalyseurs B1' et B3'.

## Revendications

1. Procédé d'hydrodésulfuration d'une coupe essence contenant des hydrocarbures ayant au moins 2 atomes de carbone par molécule et ayant un point d'ébullition final inférieur ou égal à 250°C, ledit procédé consistant en la mise en contact de ladite coupe essence avec au moins un catalyseur dont la phase active comprend au moins un métal du groupe VIII et au moins un métal du groupe VIB déposés sur un support formé d'au moins un oxyde, ledit catalyseur étant préparé selon un procédé comprenant au moins :
i) au moins une étape de mise en contact d'au moins dudit support avec au moins une solution contenant au moins un précurseur d'au moins dudit métal du groupe VIII et au moins un précurseur d'au moins dudit métal du groupe VIB,
ii) au moins une étape de mise en contact d'au moins dudit support avec au moins un composé organique formé d'au moins un oligosaccharide cyclique composé d'au moins 6 sous-unités glucopyranose liées en α-(1,4),
iii) au moins une étape de séchage pour obtenir au moins ledit métal du groupe VIII et au moins ledit métal du groupe VIB sous forme oxyde, puis
iv) au moins une étape de sulfuration de sorte que ladite phase active se présente sous forme sulfure,
les étapes i) et ii) pouvant être réalisées séparément, dans un ordre indifférent, ou simultanément.

2. Procédé d'hydrodésulfuration selon la revendication 1 tel que ladite coupe essence est issue d'une unité de craquage catalytique en lit fluidisé.

3. Procédé d'hydrodésulfuration selon la revendication 1 ou la revendication 2 tel que ladite coupe essence présente une teneur pondérale en soufre comprise entre 200 et 5000 ppm.

4. Procédé d'hydrodésulfuration selon l'une des revendications 1 à 3 tel que la phase active dudit catalyseur comprend du phosphore.

5. Procédé d'hydrodésulfuratlon selon l'une des revendications 1 à 4 tel que le métal du groupe VIB est le molybdène ou le tungstène ou un mélange de ces deux éléments.

6. Procédé d'hydrodésulfuration selon l'une des revendications 1 à 5 tel que le métal du groupe VIII est le cobalt ou le nickel ou un mélange de ces deux éléments.

7. Procédé d'hydrodésulfuration selon l'une des revendications 1 à 6 tel que ledit support est formé d'un solide poreux sous forme oxyde choisi dans le groupe constitué par les silices, les alumines de transition et les silices-alumines.

8. Procédé d'hydrodésulfuration selon l'une des revendications 1 à 7 tel que ledit composé organique est choisi parmi les cyclodextrines, les cyclodextrines substituées, les cyclodextrines polymérisées et les mélanges de cyclodextrines.

9. Procédé d'hydrodésulfuration selon la revendication 8 tel que les cyclodextrines sont l'α-cyclodextrine, la β-cyclodextrine et la γ-cyclodextrine respectivement composée de 6, 7 et 8 sous-unités glucopyranose liées en α-(1,4).

10. Procédé d'hydrodésulfuration selon la revendication 8 tel que les cyclodextrines substituées sont l'hydroxypropyl bêta-cyclodextrine et les bêta-cyclodextrines méthylées.

11. Procédé d'hydrodésulfuration selon l'une des revendications 1 à 10 tel que ladite étape iii) de séchage est réalisée à une température comprise entre 80 et 160°C.

12. Procédé d'hydrodésulfuration selon l'une des revendications 1 à 11 tel que ladite étape iii) de séchage est suivie d'au moins une étape de calcination.

13. Procédé d'hydrodésulfuration selon l'une des revendications 1 à 12 tel qu'il est mis en oeuvre dans les conditions opératoires suivantes : une température comprise entre 200 et 400°C, une pression totale comprise entre 1 et 3,5 MPa avec un rapport volume d'hydrogène sur volume de coupe essence compris entre 100 et 600 litres par litre et une vitesse volumique horaire (VVH) comprise entre 1 et 10 h⁻¹ (rapport du débit volumique de coupe essence liquide sur le volume de catalyseur chargé dans le réacteur).

14. Procédé d'hydrodésulfuration selon l'une des revendications 1 à 13 tel que ladite coupe essence contient des hydrocarbures ayant au moins 5 atomes de carbone par molécule.

## Patentansprüche

1. Verfahren zur Hydroentschwefelung einer Benzinfraktion, die Kohlenwasserstoffe enthält, die mindestens 2 Kohlenstoffatome pro Molekül aufweisen, mit einem endgültigen Siedepunkt kleiner oder gleich 250 °C, wobei das Verfahren darin besteht, die Benzinfraktion mit mindestens einem Katalysator in Kontakt zu bringen, dessen aktive Phase mindestens ein Metall aus der Gruppe VIII und mindestens ein Metall aus der Gruppe VIB umfasst, die auf einen Träger aufgebracht sind, der aus mindestens einem Oxid gebildet ist, wobei der Katalysator nach einem Verfahren präpariert ist, umfassend mindestens:
i) mindestens einen Schritt der Kontaktherstellung zwischen mindestens dem Träger und mindestens einer Lösung, die mindestens einen Vorläufer mindestens des Metalls der Gruppe VIII und mindestens einen Vorläufer mindestens des Metalls der Gruppe VIB enthält,
ii) mindestens einen Schritt der Kontaktherstellung zwischen mindestens dem Träger und mindestens einer organischen Verbindung, die von mindestens einem zyklischen Oligosaccharid, bestehend aus mindestens 6 bei α-(1,4) gebundenen Glukopyranose-Untereinheiten, gebildet ist,
iii) mindestens einen Trocknungsschritt, um mindestens das Metall der Gruppe VIII und mindestens das Metall der Gruppe VIB in Oxidform zu erhalten, dann
iv) mindestens einen Schwefelungsschritt, so dass die aktive Phase in Schwefelform vorhanden ist,
wobei die Schritt i) und ii) getrennt in beliebiger Reihenfolge oder gleichzeitig erfolgen können.

2. Verfahren zur Hydroentschwefelung nach Anspruch 1, bei dem die Benzinfraktion aus einer katalytischen Krackingeinheit im Fließbett stammt.

3. Verfahren zur Hydroentschwefelung nach Anspruch 1 oder Anspruch 2, bei dem die Benzinfraktion einen gewichtsbezogenen Schwefelgehalt zwischen 200 und 5000 ppm aufweist.

4. Verfahren zur Hydroentschwefelung nach einem der Ansprüche 1 bis 3, bei dem die aktive Phase des Katalysators Phosphor umfasst.

5. Verfahren zur Hydroentschwefelung nach einem der Ansprüche 1 bis 4, bei dem das Metall der gruppe VIB Molybdän oder Wolfram oder ein Gemisch dieser beiden Elemente ist.

6. Verfahren zur Hydroentschwefelung nach einem der Ansprüche 1 bis 5, bei dem das Metall der Gruppe VIII Kobalt oder Nickel oder ein Gemisch dieser beiden Elemente ist.

7. Verfahren zur Hydroentschwefelung nach einem der Ansprüche 1 bis 6, bei dem der Träger von einem porösen Feststoff in Oxidform gebildet ist, der in der Gruppe, die von den Siliziumoxiden, den Übergangsaluminiumoxiden und den Silizium-Aluminium-Oxiden gebildet ist, ausgewählt ist.

8. Verfahren zur Hydroentschwefelung nach einem der Ansprüche 1 bis 7, bei dem die organische Verbindung unter den Zyklodextrinen, den substituierten Zyklodextrinen, den polymerisierten Zyklodextrinen und den Gemischen von Zyklodextrinen ausgewählt ist.

9. Verfahren zur Hydroentschwefelung nach Anspruch 8, bei dem die Zyklodextrine die α-Zyklodextrine, das β-Zyklodextrin und das γ-Zyklodextrin sind, die jeweils aus 6, 7 und 8 bei α-(1,4) gebundenen Glukopyranose-Untereinheiten bestehen.

10. Verfahren zur Hydroentschwefelung nach Anspruch 8, bei dem die substituierten Zyklodextrine das Hydroxypropyl beta-zyklodextrin und die methylierten Beta-zyklodextrine sind.

11. Verfahren zur Hydroentschwefelung nach einem der Ansprüche 1 bis 10, bei dem der Schritt iii) des Trocknens bei einer Temperatur zwischen 80 und 160 °C erfolgt.

12. Verfahren zur Hydroentschwefelung nach einem der Ansprüche 1 bis 11, bei dem auf den Schritt iii) des Trocknens mindestens ein Kalzinierungsschritt folgt.

13. Verfahren zur Hydroentschwefelung nach einem der Ansprüche 1 bis 12, das unter den folgenden Betriebsbedingungen eingesetzt wird: einer Temperatur zwischen 200 und 400 °C, einem Gesamtdruck zwischen 1 und 3,5 MPa mit einem Verhältnis Sauerstoffvolumen zu Volumen der Benzinfraktion zwischen 100 und 600 Liter pro Liter und einer stündlichen Volumengeschwindigkeit (VVH) zwischen 1 und 10 h⁻¹ (Verhältnis der Volumenmenge der flüssigen Benzinfraktion zu dem in den Reaktor geladenen Katalysatorvolumen).

14. Verfahren zur Hydroentschwefelung nach einem der Ansprüche 1 bis 13, bei dem die Benzinfraktion Kohlenwasserstoffe mit mindestens 5 Kohlenstoffatomen pro Molekül enthält.

## Claims

1. A process for the hydrodesulphurization of a gasoline cut containing hydrocarbons containing at least 2 carbon atoms per molecule and having an end point of 250°C or less, said process consisting of bringing said gasoline cut into contact with at least one catalyst the active phase of which comprises at least one metal from group VIII and at least one metal from group VIB deposited on a support formed from at least one oxide, said catalyst being prepared using a process comprising at least:
i) at least one step for bringing at least said support into contact with at least one solution containing at least one precursor of at least said metal from group VIII and at least one precursor of at least said metal from group VIB;
ii) at least one step for bringing at least said support into contact with at least one organic compound formed from at least one cyclic oligosaccharide composed of at least 6 α-(1,4)-bonded glucopyranose subunits;
iii)at least one drying step to obtain at least said metal from group VIII and at least said metal from group VIB in the oxide form; then
iv) at least one sulphurization step such that said active phase is in the sulphide form;
the steps i) and ii) possibly being carried out separately, in any order, or simultaneously.

2. A hydrodesulphurization process according to claim 1, in which said gasoline cut originates from a fluid catalytic cracking unit.

3. A hydrodesulphurization process according to claim 1 or claim 2, in which said gasoline cut has a sulphur content in the range 200 to 5000 ppm by weight.

4. A hydrodesulphurization process according to one of claims 1 to 3, in which the active phase of said catalyst comprises phosphorus.

5. A hydrodesulphurization process according to one of claims 1 to 4, in which the metal from group VIB is molybdenum or tungsten or a mixture of said two elements.

6. A hydrodesulphurization process according to one of claims 1 to 5, in which the metal from group VIII is cobalt or nickel or a mixture of said two elements.

7. A hydrodesulphurization process according to one of claims 1 to 6, in which said support is formed from a porous solid in the oxide form selected from the group constituted by silicas, transition aluminas and silica-aluminas.

8. A hydrodesulphurization process according to one of claims 1 to 7, in which said organic compound is selected from cyclodextrins, substituted cyclodextrins, polymerized cyclodextrins and mixtures of cyclodextrins.

9. A hydrodesulphurization process according to claim 8, in which the cyclodextrins are α-cyclodextrin, β-cyclodextrin and γ-cyclodextrin respectively composed of 6, 7 and 8 α-(1,4)-bonded glucopyranose subunits.

10. A hydrodesulphurization process according to claim 8, in which the substituted cyclodextrins are hydroxypropyl beta-cyclodextrin and methylated beta-cyclodextrins.

11. A hydrodesulphurization process according to one of claims 1 to 10, in which said drying step iii) is carried out at a temperature in the range 80°C to 160°C.

12. A hydrodesulphurization process according to one of claims 1 to 11, in which said drying step iii) is followed by at least one calcining step.

13. A hydrodesulphurization process according to one of claims 1 to 12, carried out under the following operating conditions: a temperature in the range 200°C to 400°C, a total pressure in the range 1 to 3.5 MPa with a ratio of the volume of hydrogen to the volume of gasoline cut in the range 100 to 600 litres per litre and an hourly space velocity (HSV) in the range 1 to 10 h⁻¹ (ratio of the volume flow rate of the liquid gasoline cut to the volume of catalyst loaded into the reactor).

14. A hydrodesulphurization process according to one of claims 1 to 13, in which said gasoline cut contains hydrocarbons containing at least 5 carbon atoms per molecule.
